# EUROPEAN PATENT APPLICATION

(11) **EP 2 468 284 A1**
(43) Date of publication of application: **27.06.2012**
(21) Application number: 11191359.6
(22) Date of filing: 02.05.2006
(51) Int. Cl.: A61K 35/74, A61K 47/44, A61P 37/00, A23L 1/30, A23D 7/00

(54) **Method of improving immune function in mammals using Lactobacillus strains with certain lipids**

(30) Priority: 06.05.2005 US 123330
(62) Divisional of application: 06733377.3
(71) Applicant: Biogaia AB, 112 27 Stockholm (SE)
(72) Inventor: Kang, Ho-Jin, Incheon (KR); Connolly, Eamonn, 181 30 Lidingö (SE)
(74) Representative: Fagerlin, Heléne

(57) **Abstract**

A product for 3-HPA (reuterin) production in mammals, including humans, comprising 3-HPA producing *Lactobacillus reuteri* in combination with medium chain triglyceride oil.

## Description

### BACKGROUND OF THE INVENTION

### Field of the Invention

This invention relates to the use of the combination of MCT (medium chain triacylglyceride) oil with certain strains of *Lactobacillus* in products for mammals to achieve an improved immune effect.

### Description of the Related Art

In nutrition, the substance commonly called fat or oil is one of several substances which chemists and nutritionists classify as lipids. Fats are characteristically insoluble in water and dissolve only in certain solvents such as alcohol. Fats are similar in their composition to carbohydrates but provide a higher energy yield per gram. They contain somewhere between two to two and a half times more energy than carbohydrates. Unfortunately, fats are uneconomical in their use of oxygen for metabolism and are, as a result difficult to burn. The body requires fats for various processes, for example they are used in the construction of cell walls and they provide the transport mechanism for fat soluble vitamins (Vitamins A,D,E and K).

There are three classes of fats - triglycerides, cholesterol and phospholipids. Fats and oils found in food tend mainly to be triglycerides. Triglycerides are composed of glycerol which is an alcohol, and three fatty acid chains. Triglycerides make up the majority of the fats we consume and store in our body. They are classified into three distinct groups - saturated, mono-unsaturated and polyunsaturated. The classification of fats is linked to their chemical composition. Saturated fatty acids have a closed chemical structure, their carbon atoms being "saturated" with hydrogen, making it impossible for other compounds to link with them. Mono-unsaturated fatty acids have a single double bond and two free carbons to which other chemical bonds of hydrogen can occur. Finally, polyunsaturated fatty acids have two or more double bonds and have a number of free carbons to which links can be made. These double bonds make unsaturated fats more biologically active than the almost inert saturated fats.

The fatty acids which are components of triglycerides are made up of chains of carbon, oxygen, and hydrogen atoms of varying length. The length of the chains of carbon leads to these chains being classified as short chain, medium chain and long chain triglycerides. Medium chain triglycerides (MCT) have unique properties which make them more biologically available than other forms of fats (Osborn H.T. et al, Comprehensive Reviews in Food Science and Food Safety, Vol1, 2002, page 93-103). MCTs by-pass the usual route taken by fats which the body uses to make the energy available. Unlike other fats, which require the body to store them before use, MCTs require limited processing and are, as a result quickly available for use in the body's energy systems. Another significant feature of medium chain triglycerides is that the body is unable to store them as body fat no matter how much is consumed.

MCT oils occur naturally, and the most abundant source is coconut oil. Most MCT oil is refined from coconut oil. MCT oil is a clear light colored liquid with no flavor and low viscosity. MCT oil is interesting because, when it is metabolized in the body, it behaves rather more like a carbohydrate than a fat. The fuel of preference for the body is carbohydrate, and the body will use up its store of carbohydrate before using other fuels. Carbohydrates are quick acting - athletes take glucose tablets to provide energy, the body heats rapidly when we drink alcohol - typically carbohydrates will be used within a few hours of eating, which is why we eat so frequently. By contrast, the primary role of fats is to store energy - animals fatten up to prepare for the rigor of winter. Long chain fats (i.e., the normal varieties) are converted into chemicals called chylomicrons by the digestive system, and these are then transported around the body by the lymphatic system before entering the circulatory system. This is a relatively slow process, and so fats metabolize more slowly than carbohydrates. Unlike other fats, MCT oil does not go into the lymphatic system; instead it is transported directly to the liver where it is metabolized, so releasing energy quickly, just like a carbohydrate, and creating many ketones in the process. MCT is commonly used in various nutritional products, including medical nutrition, such as pareneteral or enteral nutrition.

MCTs as such have also been reported to have more direct health effects. For example Tufano M. A. et al. in "Survival to lipopolysaccharide, cytokine release and phagocyte functions in mice treated with different total parenteral nutrition regimens". (Immunopharmacol Immunotoxicol. 1995 Aug;17(3):493-509) reported on effects on host defenses of Total Parenteral Nutrition (TPN) with long- (LCT) and medium-chain triglycerides (MCT). Survival after lipopolysaccharide (LPS) challenge, blood clearance of *Escherichia coli, in vivo* and *in vitro* production of tumor necrosis factor-alpha (TNF-alpha) and interleukin-6 (IL-6) were investigated. In BALB/c mice, LCTs produced a 25% reduction in mortality, compared with controls. TPN performed with a LCT plus MCT mixture reduced mortality by 50%.

Clinically fine control of the concentration of injected triglycerides, especially MCT, can be expected to provide potent antitumor effect and maintenance of the normal immune system (Kimoto Y. et al.; Antitumor effect of medium-chain triglyceride and its influence on the self-defense system of the body; Cancer Detect Prev. 1998;22(3):219-24). MCT oil as such is also mentioned as part of nutritional formulations for example in European patent EP0756827 and US patent US6589576, but not in combination with any *Lactobacillus.* Further, the European patent application EP 1 344 458 A1 discloses a concept of protecting probiotic organsisms such as *Lactobacillus,* making pellets, preferably of a minimum volume of 0.02 cm³ and then possibly coated with various barriers, including MCT oil. This work does not come close to the core of the present invention, namely combining selected 3-HPA (ß-hydroxy-propionaldehyde) producing strains of *Lactobacillus* with MCT oil in a liquid product for improved immune effect.

A probiotic, by the generally accepted definition, is a live microbial feed supplement which beneficially affects the host animal by improving its intestinal microbial balance. Although originally referring to the supplementation of animal feeds for farm animals, the definition is easily applied to the human situation. The major consumption of probiotics by humans is in the form of dairy-based foods containing intestinal species of lactobacilli and bifidobacteria. It is implicit in the definition that consumption of the probiotic affects the composition of the intestinal microflora. This effect of the probiotic on the intestinal ecosystem, it is proposed, impacts in some beneficial way on the consumer. A number of potential benefits arising from changes to the intestinal milieu through the agency of probiotics have been documented including: increased resistance to infectious diseases, particularly of the intestine, decreased duration of diarrhea, reduction in blood pressure, reduction in serum cholesterol concentration, reduction in allergy, stimulation of phagocytosis by peripheral blood leucocytes, modulation of cytokine gene expression, adjuvant effect, regression of tumours, and reduction in carcinogen or co-carcinogen production.

Strains of a wide variety of *Lactobacillus* species, including *L. reuteri* have been used in probiotic formulations. *Lactobacillus reuteri* is one of the naturally occurring inhabitants of the gastrointestinal tract of animals, and is routinely found in the intestines of healthy animals, including humans. It is known to have antimicrobial activity. See, for example U.S. Patent Nos. 5,439,678, 5,458,875, 5,534,253, 5,837,238, and 5,849,289. When *L. reuteri* cells are grown under anaerobic conditions in the presence of glycerol, they produce the antimicrobial substance known as ß-hydroxy-propionaldehyde (3-HPA).

3-HPA is a metabolic intermediate that is secreted from the cell by a few lactobacilli. Those bacterial species known to export 3-HPA include *L. reuteri, L. coryneformis L. collinoides, L. hilgardii* etc. (Claisse O et. al. J Food Prot. 2001 Jun;64(6):833-7) (SauvageotN et. al. Int J Food Microbiol. 2000 Apr 10;55(1-3):167-70). 3-HPA has long been known to have antimicrobial properties which partially explain the ability of the producing strains to kill pathogenic bacteria. Lactic acid bacteria, including *L. reuteri* and *L. coryneformis,* have also been shown to have influence on the immune system of their host organism. See, for example "Biotherapeutic effects of probiotic bacteria on candidiasis in immunodeficient mice" by Wagner RD, et al., (Infect Immune 1997 Oct 65:4165-72); however, differences in efficacy exists between strains and methods are needed to increase the effects, for example the method selecting strains recruiting CD4+ cells and binding toxins, provided in WO 2004/034808. The exact mechanisms through which stimulation or modification of the activity of the host immune cells by lacticobacilli is still mostly unclear. Many studies indicate that specific substances derived from selected lactic acid bacteria that are released into the growth medium are responsible for modulating the immune response of host cells. These substances are generally believed to be proteins, peptides and nucleic acids. See, for example, Pena et al, Cell Microbiol. 2003 Apr;5(4):277-85.

The invention herein provides a new method for improving the immunomodulatory effects of certain lactobacilli in some animals, including humans, namely by combining selected 3-HPA producing *Lactobacillus* with a MCT oil. The effect of this combination is in addition to previously known immune-modulation and antimicrobial effects by those lactobacilli. It is caused by their production of 3-HPA and the direct effect on the proliferation of the lymphocytes by this substance. 3-HPA or its precursor glycerol, or its metabolites, 1,3 propanediol and 3-OH-propionic acid, have not earlier been described as potential modulators of the immune system in mammals.

While the possibility of effective antimicrobial activity by several lactobacilli is known, and certain immunomodulatory effects were also known, it was not previously known that substantial improvements of immunomodulatory effects was possible in 3-HPA producing strains by combining them with MCT oil.

It is therefore an object of the invention to provide strains of *Lactobacillus* which are known to produce 3-HPA under the proper conditions and to combine them with MCT oil for improved immunomodulation. It is a further object of the invention to provide products containing said strains and MCT oil for the administration to animals, including humans.

Other objects and advantages will be more fully apparent from the following disclosure and appended claims.

### SUMMARY OF THE INVENTION

The invention herein is a method of improving immune-function in mammals using selected 3-HPA producing *Lactobacillus* with a MCT oil. The effect of this combination is in addition to previously known immune-modulation and antimicrobial effects by those lactobacilli. It is caused by their production of 3-HPA and the direct effect on the proliferation of the lymphocytes by this substance. Other objects and features of the inventions will be more fully apparent from the following disclosure and appended claims.

### BRIEF DESCRIPTION OF THE DRAWINGS

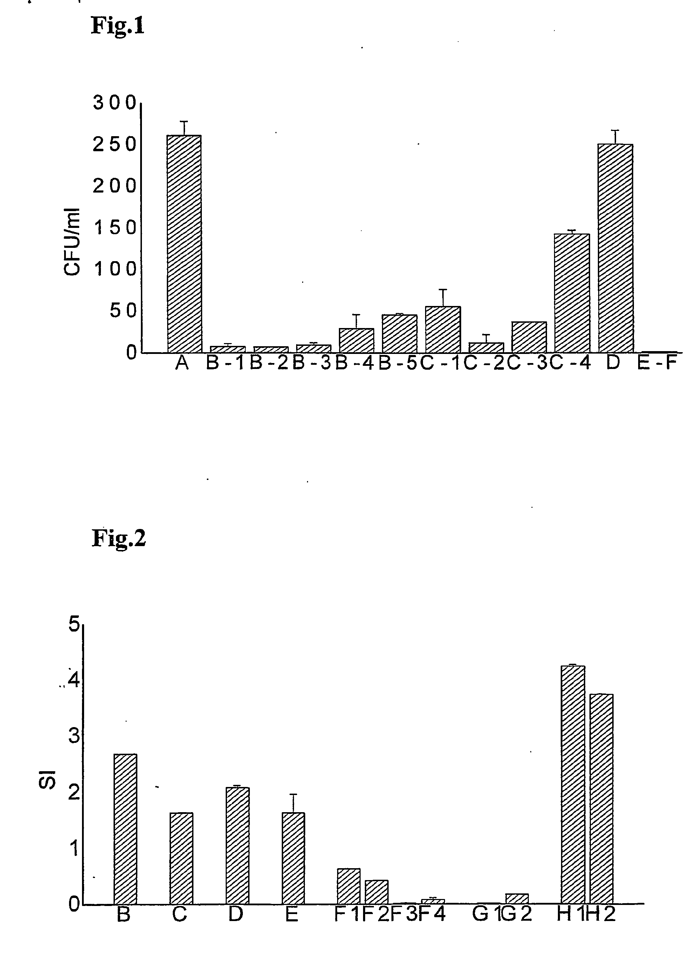

Figure 1 shows the results of a phagocytic assay: A, Cell control; B1, *L. reuteri* lysate (800 µg/ml); B2, *L. reuteri* lysate (400 µg/ml); B3, *L. reuteri* lysate (200 µg/ml); B4, *L. reuteri* lysate (100 µg/ml); B5, *L. reuteri* lysate (50 µg/ml); C1, *L. monocytogenes* lysate (800 µg/ml); C2, *L. monocytogenes* lysate (400 µg/ml); C3, *L. monocytogenes* lysate (200 µg/ml); C4, *L. monocytogenes* lysate (100 µg/ml); D, *L. reuteri* supernatant (MRS); E, *L. reuteri* supernatant (glycerol sol); F, *L. reuteri* +*L. monocytogenes* lysates.
Figure 2 shows the results of a lymphocyte proliferation assay: B, Con A (6.25 µg/ml); C, PWM (5 µg/ml); D, PHA (6.25 µg/ml); E, LPS (2.5 µg/ml); F1, *L. reuteri* lysate (400 µg/ml); F2, *L. reuteri* lysate (200 µg/ml); F3, *L. reuteri* lysate (100 µg/ml); F4, *L. reuteri* lysate (50 µg/ml); G1, *L. reuteri* supernatant (MRS 100 µl); G2, *L. reuteri* supernatant (MRS 50 µl); H1, *L. reuteri* supernatant (glycerol sol. 100 µl); H2, *L. reuteri* supernatant (glycerol sol. 50 µl).

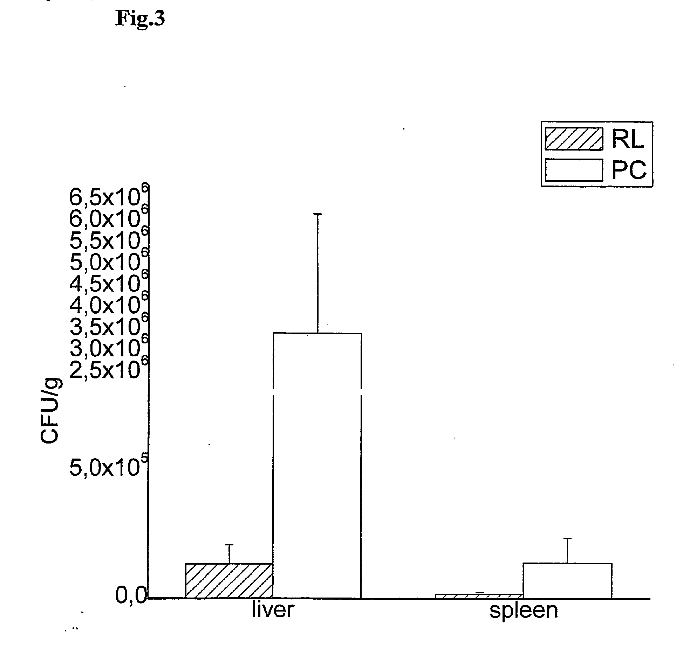

Figure 3 shows the bacterial counts at 3 days after administration of *L. monocytogenes*(6d) in the RL (*L. reuteri*) and PC (positive control) treatments.

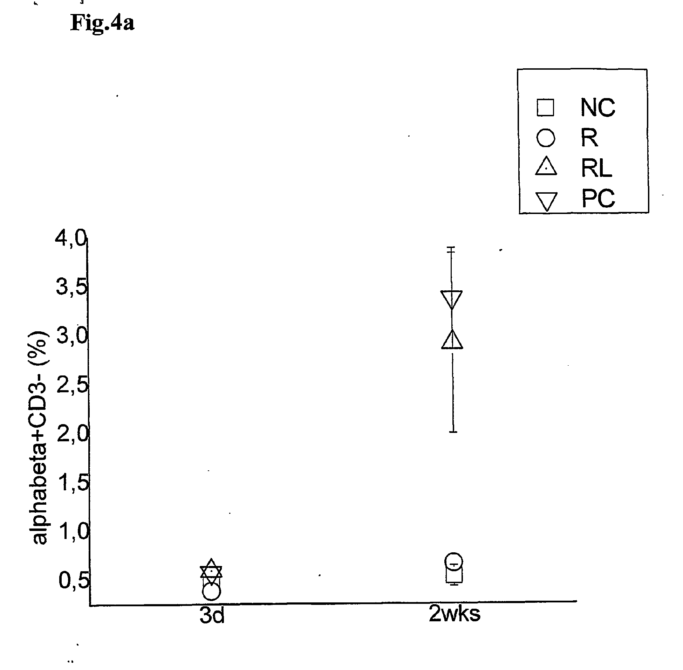

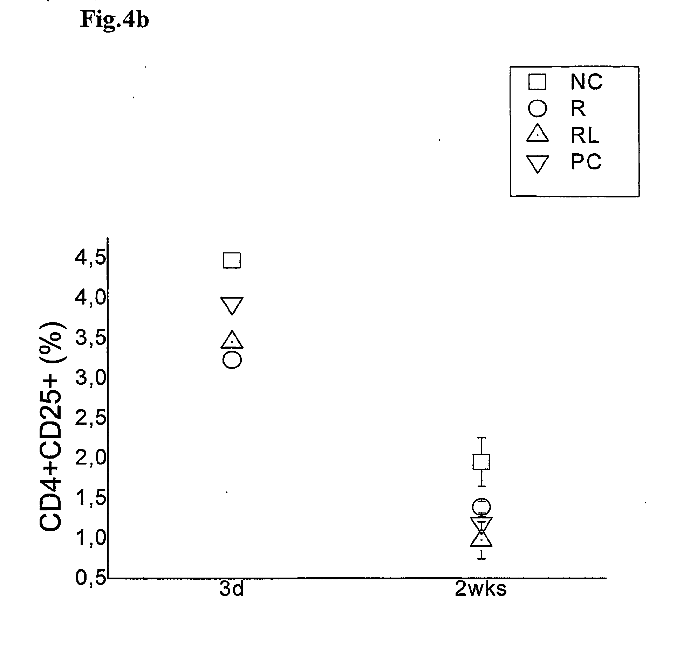

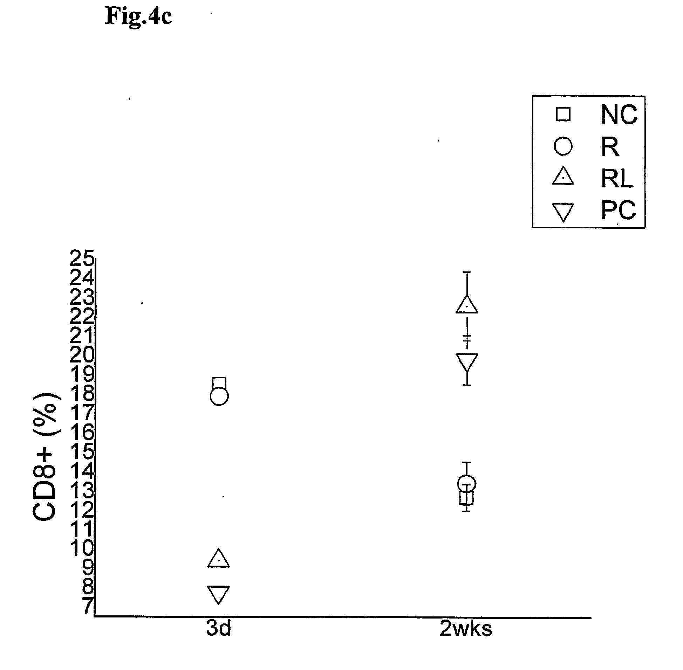

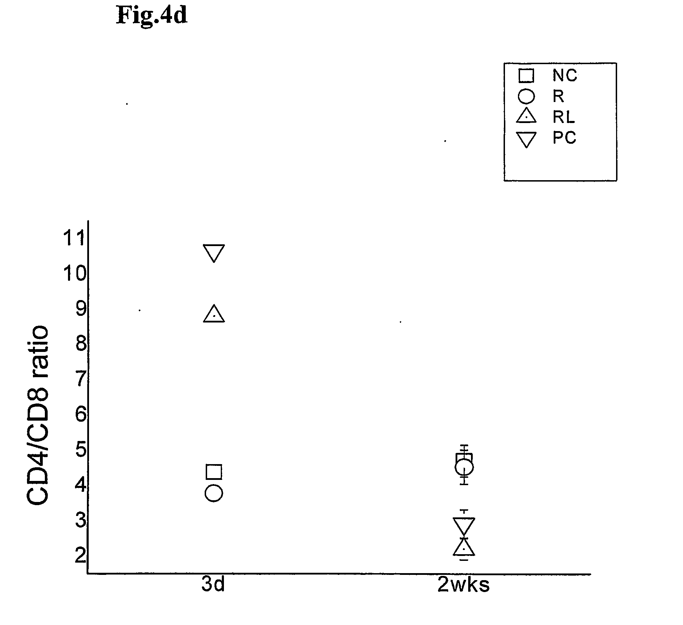

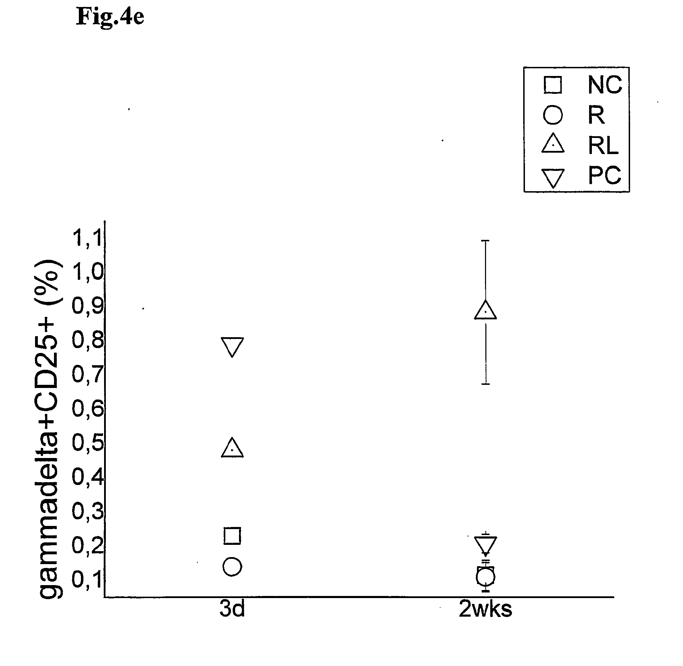

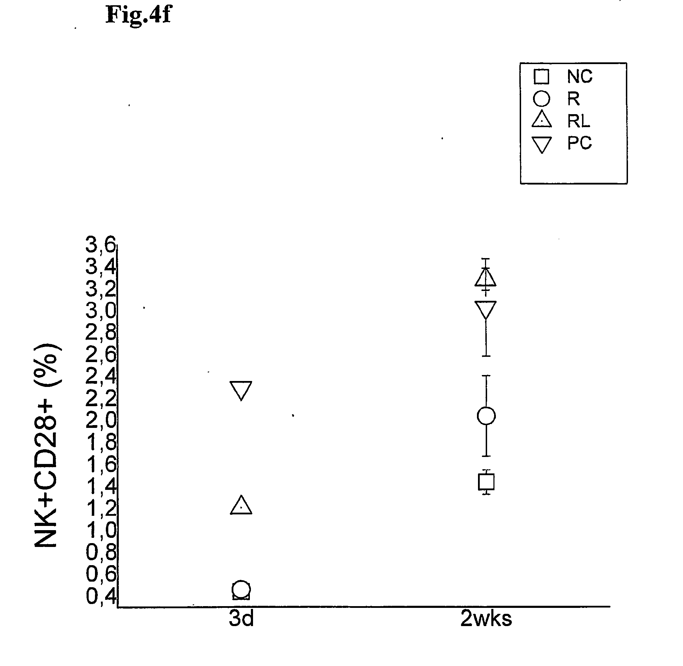

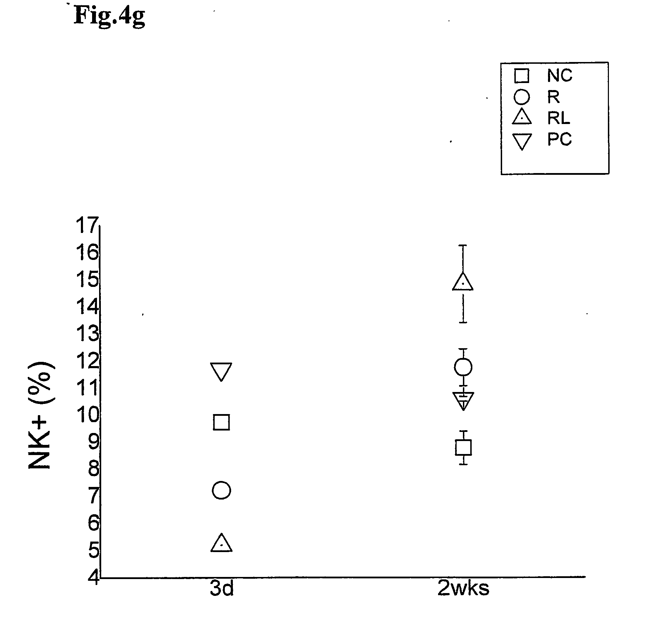

Figures 4a-4g show the results of analyzing major immune cells at 3 days (6d) and 2 weeks (17d) after inoculation *of L. monocytogenes.* In particular, Figure 4a shows (beta)TCR+CD3- T cells, Figure 4b shows the CD4+CD25+ T cells, Figure 4c shows CD8+ T cells, Figure 4d shows the CD4/CD8 ratio, Figure 4e shows (gamma, delta)TCR+CD25+ T cells, Figure 4f shows NK+CD28+ cells, and Figure 4g shows NK+ cells.

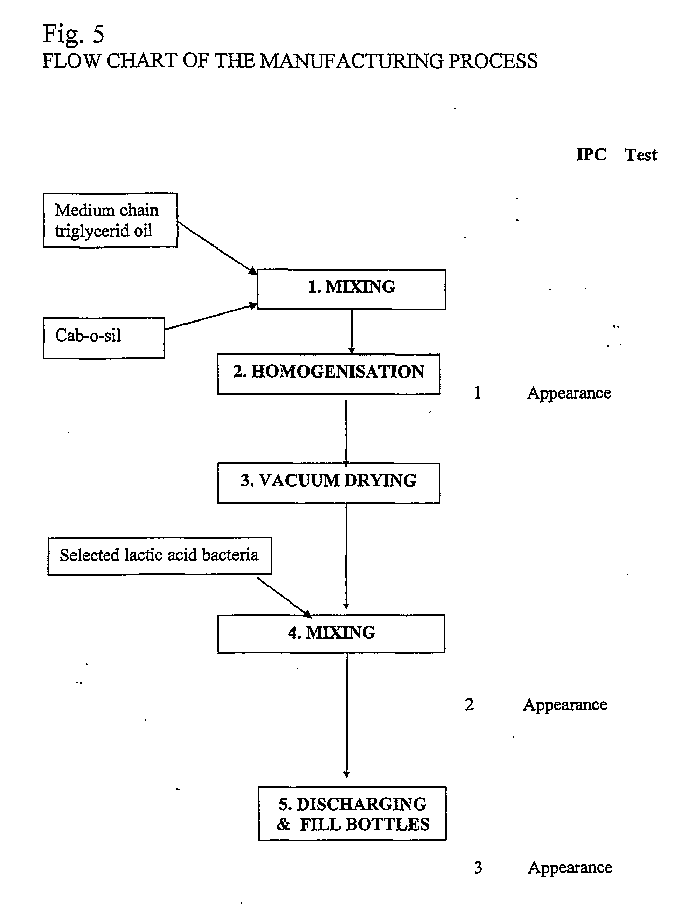

Figure 5 is a flow chart showing an example of the manufacturing process for the product of the invention.

### DETAILED DESCRIPTION OF THE INVENTION AND PREFERRED EMBODIMENTS THEREOF

Lactic acid bacteria that produce and export substances or metabolites in response to incubation with glycerol were surprisingly found to stimulate important cellular components of the immune system consistent with an overall improvement of the host's immune system and ability to combat infections. The feeding of substrates (such as oils, fats and lipids) and conditions that can be expected to generate glycerol through metabolism in the GI tract in combination with such lactic acid bacteria producing 3-HPA from glycerol, stimulates the immune system of the host. MCT oils are most suitable as such additives of the invention herein, as more described below. Thus it is advantageous to deliver such lactic acid bacteria to the host in such formulations.

One preferred embodiment of the invention is a product, containing MCT oil and 3-HPA producing lactic acid bacteria, which is formulated and used for oral delivery or tube-feeding to the GI tract of people with under-developed fat metabolism, for example most normal infants and toddlers, but also adults with fat metabolism disturbances. The reason is that fat metabolism, of, for example, the MCT-oil of the present invention, does not normally generate glycerol in the GI-tract but rather monoacylglycerols (glycerol with one fatty acid still attached). These monoacyl-glycerols are taken up over the gut wall and processed to new lipids in the liver and circulation; however, for example, the bile salt-stimulated lipase secreted with human milk was found to be devoid of positional specificity, i.e., it hydrolyzed emulsified triacylglycerols to glycerol and fatty acids. It also hydrolyzed micellar sn-2 monoacylglycerols ("sn" refers to the position of the fatty acid on the glycerol backbone of the acylglycerol, with the sn-2 position, for example, having the fatty acid is in the middle position of the glycerol backbone). This is in contrast to pancreatic lipase which has a pronounced preference for hydrolysis of sn-1 and sn- 3 ester bonds. When the two enzymes are operating together, as in the intestine of the infant fed raw human milk, the sn-2 monoacylglycerols formed by pancreatic lipase served as an excellent substrate for bile salt-stimulated lipase. (Pediatric Research, Vol 16, 882-885, Digestion of human milk lipids: physiologic significance of sn-2 monoacylglycerol hydrolysis by bile salt-stimulated lipase, O. Hernell and L. Blackberg). Thus, the end products of triacylglycerol hydrolysis became glycerol and fatty acids and not sn-2 monoacylglycerol and fatty acids. The bile salt-stimulated lipase also catalyze incorporation of fatty acids into acylglycerols to a much lesser extent than pancreatic lipase. Together these two effects of bile salt-stimulated lipase have a promoting effect on the overall process of intraluminal lipolysis. In newborn infants, with low intraduodenal bile salt concentrations, glycerol and fatty acids also should be more readily absorbed than monoacylglycerol and fatty acids. Thus, by serving as a complement to pancreatic lipase, bile salt-stimulated lipase can ensure efficient utilization of for example milk lipids also in infants, and others, with immature endogenous mechanisms for fat digestion and absorption. Consequently, for example, infants have a different system to take up lipids from their mother's milk (and infant formulas) as their fat metabolism is immature, thus, 3-HPA producing *Lactobacillus* given as a supplement to nursing infants will in such cases have a reasonably good source of glycerol to work with, but in order to secure the availability of fat that can be metabolized to be a substrate for 3-HPA production and improved immune-function of the present invention, the idea of the invention is that a fat-source such as MCT oil should be delivered together with the 3-HPA producing *Lactobacillus.* This helps improve the action of the immune system of an infant for example.

In the above preferred embodiment, the product of the invention is delivered to the GI-tract for improvement of the immune-system, in whole or in part, of the recipient. The reason that the production of 3-HPA in the GI-tract can influence the whole of the immune-system is the abundance of immunological functions in the GI-tact. This is through, for example, cells such as macrophages and lymphocytes that are growing in the mucosa and the gut lining. The GI-tract is considered by many experts in the field to be the largest immune-organ of the human body ("Overview of gut flora and probiotics", Holzapfel, W.H. et. al., International Journal of Food Microbiology, May 1998).

In another preferred embodiment of the invention a similar product may be used, containing for example MCT-oil and 3-HPA producing lactic acid bacteria, but for topical application to enhance the immune-system and combat skin disorders. The diffusion through the skin epithelium and interaction with immune cells underneath is expected for the improved immune-functions against pathogens as well as for reduced inflammation and healing, e.g. in eczema, acne and wounds.

MCT oils are unusual lipids- they are cleaved rapidly and can give rise to glycerol quickly- this is why MCT is used in this invention to give an improved immune effect. The probiotic, prophylactic and pharmaceutical products according to the invention may comprise additives and excipients acceptable for nutritious or pharmaceutical use. Medium chain triglyceride oil is an oil comprising medium chain triglycerides i.e. medium chain fatty esters of glycerol. Medium chain fatty acids are generally known in the art and may comprise from 6 to 12 carbon atoms preferably 8 to 10 carbon atoms such as caprylic and capric acids. Akomed R from Karlshamn is one example of a useful MCT oil according to the invention. It comprises caprylic /capric triglyceride.

As can be seen from the examples below, MCT oils to be used in products of the invention are easily sourced on the market. Different methods to isolate the lactic acid bacteria producing 3-HPA can be used, for example the one described by Rodrigues E. et al. (Letters in Applied MicrobiologyVolume 37 Issue 3 Page 259 - September 2003). One of the prefered species is *L.reuteri* and one preferred strain is *L.reuteri* ATCC 55730.

The features of the present invention will be more clearly understood by reference to the following examples, which are not to be construed as limiting the invention.

### Example 1: IMPROVED PHAGOCYTIC ACTIVITY IN MACROPHAGES Summary

*L. reuteri* supernatants were derived from growing *L. reuteri* ATCC 55730 in the presence and absence of glycerol (50 mM). These supernatants were used to pretreat abdominal macrophages (derived from SPF BALB/c 6week old mice) in overnight incubations. The macrophages were then seeded with *Listeria monocytogenes* to determine their phagocytic capacity. After 2 hours incubation, the amount of live *L. monocytogenes* in the macrophages was determined.

Control incubations in the absence *of L. reuteri* supernatants showed significant survival *of L. monocytogenes* in the macrophages under these conditions. Pre-treatment of the macrophages with *L. reuteri* supernatant from cells grown in the absence of glycerol did not affect the level of phagocytosis compared to the control incubation **(****Figure 1****).** However, surprisingly, pre-treatment of the macrophages with *L. reuteri* supernatant from cells grown in the presence of glycerol led to the effective killing of all the *L. monocytogenes* in the macrophages. Thus, growth of *L. reuteri* in the presence of glycerol led to the stimulation of the phagocytic activity of the cells. Glycerol alone in the medium (control) did not have such an effect (control "H" the same as the other control).

Similar but less pronounced stimulation of macrophage phagocytic activity may be induced in a dose dependent manor by pretreating the macrophages with lyastes from *L. reuteri* or from *L. monocytogenes.* The glycerol-induced metabolism in *L. reuteri* led to a more effective stimulation of phagocytic activity, better than that induced by antigen exposure.

### DETAILS OF EXPERIMENT

### Method of study

1. Phagocytic assay and lymphocyte proliferation assay. Lymphocytes and abdominal macrophages were separated from specific pathogen-free BALB/c mice (6 weeks old) and assayed for any immune reenforcement effect in a host. Phagocytic assay using abdominal macrophages.
   - Separation of abdominal macrophages. BALB/c mice were sacrificed by cervical dislocation, then infused with 5 ml of Iscove's modified Dulbecco's medium (IMDM) and massaged. The infused IMDM was recovered, and living cells were counted by tryphan blue exclusion technique and used in the experiment at an adjusted final concentration of 1x10⁶/ml.
   - Preparation of bacterial lysates. *Lactobacillus reuteri* SD2112 and *Listeria monocytogenes* HPB3 serotype 4b were incubated in MRS+0.02 M glucose broth and Tryptic Soy Broth (TSB), respectively for 24 hours, and centrifuged for 20 minutes at 8,000 rpm in order to obtain cell pellet. The cell pellet was washed two times with 0.9% NaCl solution. Then, this pellet was inactivated by heating at 60° for 3 hours, and protein concentration was calculated by Bicinchoninic acid (BSA) protein assay method and adjusted to 5 mg/ml.
   - Preparation *of L. reuteri* supernatants. *L. reuteri* was incubated in MRS+0.02 M glucose broth for 24 hours and centrifuged in order to obtain MRS broth supernatant. The supernatant was suspended in 0.05M glycerol solution and incubated for 6 hours, and then the glycerol solution supernatant was recovered. The amount of cell pellet recovered was up to 2g/30 ml (wet weight). All of the recovered supernatant was used after adjusting the pH to 7.3.
   - Phagocytic assay. Abdominal macrophages were suspended in IMDM+10% horse serum at 4x10⁵ per well and treated with varying concentrations of *L. reuteri, L. monocytogenes* cell lysate and *L. reuteri* supernatant during overnight incubation, the concentrations and conditions of which were as follows:

**Table 1. The treatment condition for a phagocytic assay**

| No. | Treatment | (µg/ml) | No. | Treatment | (µg/ml) |
|---|---|---|---|---|---|
| A | Cell control | - | D1 | *L. reuteri* supernatant (MRS) | 100 |
| B1 | *L. reuteri* lysate | 800 | D2 | L. reuteri supernatant (MRS) | 50 |
| B2 | *L. reuteri* lysate | 400 | E1 | *L. reuteri* supernatant (Glycerol sol.) | 100 |
| B3 | *L. reuteri* lysate | 200 | E2 | *L. reuteri* supernatant (Glycerol sol) | 50 |
| B4 | *L. reuteri* lysate | 100 | F1 | *L. reuteri* +*L. monocytogenes* lysates | 400+400 |
| B5 | *L. reuteri* lysate | 50 | F2 | *L. reuteri* +*L. monocytogenes* lysates | 200+200 |
| C1 | *L. monocytogenes* lysate | 800 | F3 | *L. reuteri* +*L. monocytogenes* lysates | 100+100 |
| C2 | *L. monocytogenes* lysate | 400 | G | MRS control | 100 |
| C3 | *L. monocytogenes* lysate | 200 | H | Glycerol sol. Control | 100 |
| C4 | *L. monocytogenes* lysate | 100 | I | IMDM control | - |

MRS and glycerol sol. control were used by adjusting pH in broths which were not incubated with *L. reuteri.*

Abdominal macrophages treated in the above conditions were incubated in TBS and seeded with 4x10⁴ *L. monocytogenes* (HPB3 serotype 4b, gentamycin susceptible) washed with 0.9% NaCl solution. After incubation at 37° for 2 hours, the macrophages were washed and then treated with 200 µg/ml gentamycin to kill extracellular bacteria. Cells were washed three times with Phosphate Buffered Saline (PBS) and were lysed with 0.5 ml/well of PBS+0.5% Triton X-100 to expose intracellular microorganisms. Then, the number of bacteria was calculated with the thin layer method using Palcam agar (Oxoid Inc, Ogdensburg, N.Y., USA) and Tryptic Soy agar (Oxoid Inc, Ogdensburg, N.Y., USA) following serial dilution.

### Example 2: Improved lymphocyte proliferation

### Summary

Lymphocytes were prepared from the spleen and thymus of BALB/c mice and were incubated with supernatants derived from growing *L. reuteri* ATCC 55730 in the presence and absence of glycerol (50 mM). *L. reuteri* ATCC 55730 was selected as it is a known good producer of 3-HPA and thus follows the selection criteria of the invention. Control incubations were performed with glycerol alone, media alone and known lymphocyte proliferation stimulants.

Known stimulants (ConA, PWM, PHA abd LPS) led to a marked stimulation of lymphocyte proliferation. Lysates from *L. reuteri* led to a small, dose dependent stimulation of proliferation of the lymphocytes, and incubation with supernatants from *L. reuteri* grown in the absence of glycerol led to no significant elevation in lymphocyte proliferation. Surprisingly, supernatants from *L. reuteri* grown in the presence of glycerol led to a pronounced proliferation of the lymphocytes to a level higher than that seen with known stimulants (see fig. 2).

### Details of Experiment

### Lymphocyte proliferation assay

- Separation of spleen and thymus lymphocytes. BALB/c mice were sacrificed by cervical dislocation, and their thymus was separated, immersed in Hanks' balanced salt solution (HBSS) and torn piecemeal to obtain a single cell. The single cell was centrifuged through double layers in Histopaque-1083 (Sigma) to obtain lymphocytes. These cells were washed two times with IMDM, and living cells were counted according to tryphan blue exclusion technique and used in the experiment at an adjusted final concentration of 1 x 10⁷/mL.
- Preparation of bacterial lysates. *L. reuteri* and *Listeria monocytogenes* lysates were prepared as mentioned in the phagocytic assay.
- Preparation of *L. reuteri* supernatants. *L. reuteri* supernatant was also prepared as mentioned in the phagocytic assay.
- Lymphocyte proliferation assay. Lymphocyte proliferation assay was carried out using lymphocytes suspended in IMDM+10% horse serum at 4x10⁶ per well. The lymphocytes were treated with varying concentrations of *L. reuteri, L. monocytogenes* cell lysate and *L. reuteri* supernatant along with a reference stimulant, of which concentrations and conditions were as follows:

**Table 2. The treatment conditions for a lymphocyte proliferation assay**

| No. | Treatment | (µg/ml) | No. | Treatment | (µg/ml) |
|---|---|---|---|---|---|
| A | Cell control | - | G1 | *L. reuteri* supernatant (MRS) | 100 |
| B | ConA | 6.25 | G2 | *L. reuteri* supernatant (MRS) | 50 |
| C | PWM | 5 | H1 | *L. reuteri* supernatant (Glycerol sol.) | 100 |
| D | PHA | 6.25 | H2 | *L. reuteri* supernatant (Glycerol sol.) | 50 |
| E | LPS | 2.5 | I | IMDM control | - |
| F1 | *L. reuteri* lysate | 400 | J1 | MRS control | 100 |
| F2 | *L. reuteri* lysate | 200 | J2 | MRS control | 50 |
| F3 | *L. reuteri* lysate | 100 | K1 | Glycerol sol. Control | 100 |
| F4 | *L. reuteri* lysate | 50 | K2 | Glycerol sol. Control | 50 |

In the results reported in Table 2, MRS and glycerol sol. controls were used by adjusting pH in broths which were not incubated with *L. reuteri,* and the following symbols used: ConA: concanavalin A, PWM: pokeweed mitogen, PHA: phytohemaaglutinin, LPS: liphopholysaccharide from *Salmonella enterica* serovar Typhimurium.

Stimulants including ConA were added into a well containing lymphocytes in accordance with each condition, 3 hours before alamar blue was added at 10% concentration. 72 hours after the addition of each stimulant, the level of lymphocyte proliferation was determined by the value of OD570 nm-OD600 nm.

The result of lymphocyte proliferation was represented as Stimulation Index (SI), which is (ODtreatment-ODmedia control)/ODcell control value. In the case of *L. reuteri* supernatant treatment group, however, values of J and K treatment groups, but not IMDM+10% horse serum, were used as OD media control value.

### Mouse in vivo challenge and change of distribution rate in immune cells

Specific pathogen free BALB/c mice (6 weeks old) were divided into four groups. *L. reuteri* and *L. monocytogenes* were prepared to 5x10⁸/mouse and orally administered. Thirty minutes before the time of administrating *L. monocytogenes,* 10% sodium bicarbonate 0.25M was administered so as to improve the survival rate of *L. monocytogenes.*

**Table 3. Mouse treatment groups**

| Group | Treatment | Sampling |
|---|---|---|
| Negative control (NC) | PBS administration in the study period | 6d, 17d, 31d |
| R | *L. reuteri* administration in the study period | 6d, 17d |
| RL | *L. reuteri* administration in the study period. *L. monocytogenes* administration at 3d | 6d, 17d |
| Positive control (PC) | PBS administration in the study period. *L. monocytogenes* administration at 3d | 6d, 17d, 31d |

In order to investigate the protective effect *of L. reuteri* against *L. monocytogenes,* the respective 8 mice of every group were sacrificed at 3 days (6d) and 14 days (17d) after administration of *L. monocytogenes,* from which blood, liver and spleen were taken. In addition, in order to investigate the involvement of *L. reuteri* itself in immune mechanisms, the respective 8 mice of both NC group and R group were further assayed at 31d.
1) Calculation *of L. monocytogenes* in liver and spleen. The liver and spleen taken from each mouse group were weighted and homogenized in a whirl-pack containing 10 ml of PBS (pH 7.0). Living bacteria was counted by Palcam agar, following serial dilution.
2) Immune cell assay using flow cytometry
   - Separation of leukocytes from blood. Blood was drawn, pooled by 2-3 animals and centrifuged through double layers in Histopaque-1083 at 1,500 rpm for 30 minutes, after which lymphocytes were gathered from lymphocyte layers. They were washed three times with phosphate buffered saline (PBS) and suspended RPMI-1640 media, then living cells were counted according to tryphan blue exclusion technique and used in the experiment at an adjusted final concentration of 1x10⁷/ml.
   - Flow cytometry assay using leukocyte-surface monoclonal antibodies. The separated leukocytes were suspended into the first washing buffer (PBS 450 ml, ACD 50 ml, 20% NaN₃ 5 ml, gamma globulin free horse serum 10 ml, 250 mM EDTA 20 ml, 0.5% phenol red 1 ml) containing 20% goat serum, and sensitized at 4°C for 10 minutes. After centrifugation, to each well of V-bottomed 96 well-microplates were added 100 µl of monoclonal antibodies specific for leukocyte-surface antigens and 1x10⁷/ml of 50 µl leukocytes separated from blood, prior to sensitization at 4°C for 30 minutes. Dual staining was carried out, in which reagents used were the same as in Table 4. After washing three times with a first washing buffer, the remaining leukocytes were washed three times with 4°C of a second washing buffer which was the same as the first washing buffer except that horse sera were removed, and fixed in 2% formalin solution. More than 2,000 of the stained cells were inspected by flow cytometry, and data analysis was performed by means of Cell Quest program (Becton Dickinson).

**Table 4. Monoclonal Abs used in an immune cell assay**

| Marker | (alpha beta) TCR/CD3 | CD4/CD25 | CD8a/CD25 | (gamma delta) TCR/CD25 | NK(CD49b)/C D28 |
|---|---|---|---|---|---|
| Fluorescence | FITC conjugated rat anti-mouse (alpha beta) TCR | FITC conjugated rat anti-mouse CD4 | FITC conjugated rat anti-mouse CD8a | FITC conjugated hamster anti-mouse (gamma delta) TCR | FITC conjugated rat anti-mouse CD49b/PAN-NK |
| Fluorescence | PE conjugated rat anti-mouse CD3 | PE conjugated rat anti-mouse CD25 | PE conjugated rat anti-mouse CD25 | PE conjugated rat anti-mouse CD25 | PE conjugated hamster anti-mouse CD28 |

### Result and Discussion

### 1) Phagocytic assay using abdominal macrophages

The results of a phagocytic assay using abdominal macrophages are shown in **F**igure 1. As shown, the result of a phagocytic assay of abdominal macrophages was confirmed to be highest in cases of treatment with *L. reuteri* glycerol solution supernatant and with *L. reuteri* + *L. monocytogenes* lysates (P<0.05). In other words, *L. reuteri* glycerol solution supernatant had an identical effect regardless of 100 µl or 50 µl treatment, and almost completely killed *L. monocytogenes* that had been phagocytosed by the macrophages. Mixed treatment of each 400, 200 and 100 µl/ml *of L. reuteri + L. monocytogenes* lysates also practically killed *L. monocytogenes* that had been phagocytosed by the macrophages. The group treated with *L. reuteri* lysates alone had less phagocytic activity than the above 2 treatment groups and *L. monocytogenes* were living even after phagocytosis, though the phagocytic activity of *L. reuteri* lysates treatment group (B) was seen to be higher than that of *L. monocytogenes* lysate treatment group (C)(P<0.05). In light of these results, it was recognized that the *L. reuteri* lysate itself and its metabolites could enhance the phagocytic activity of abdominal macrophages, especially in the presence of pathogen lysate, for example *L. monocytogenes.* Taking into consideration that the group treated with MRS broth supernatant was found to be unable to enhance the phagocytic activity of abdominal macrophages; whereas the group treated with glycerol solution supernatant showed high phagocytic activity, the material to enhance phagocytic activity is presumed to be glycerol metabolites and cannot be presumed to rule out the possibility of being reuterin with high antibiotic ability. This issue is thought to need further study through subsequent experiments.

The group treated with *L. monocytogenes* lysate was also found to have somewhat enhanced phagocytic activity, though its activity was less than that of *L. reuteri* lysate treatment groups. It should be considered that this result was not the result obtained by treatment with living *L. monocytogenes,* and the noticeable particular is that phagocytic activity was confirmed to be rather decreased in the group treated with a high concentration *of L. monocytogenes* lysate. It seems this is the result of the vitality of macrophages to be inhibited by several virulent factors present in high concentrations *of L. monocytogenes* lysate.

### 2) Lymphocyte proliferation assay

The results shown in **Figure 2** were achieved by a lymphocyte proliferation assay using lymphocytes separated from spleen and thymus. Proliferation of lymphocytes separated from the spleen and thymus was confirmed to be highest in *L. reuteri* supernatant (glycerol sol.) treatment group (P<0.05).

A method of measuring proliferation used in the experiment is an alamar blue assay, the principle of which lies in the extent of reduction of reagents due to cell proliferation. It cannot be ruled out that its value may have been measured high by reagent reduction due to aldehyde oxidization by reason that *L. reuteri* supernatant (glycerol sol.) contains 3HPA, an aldehyde. However, it is determined that since cell proliferation *of L. reuteri* supernatant (glycerol sol.) treatment group was confirmed to be the highest under microscopic observation too, lymphocyte-proliferative ability *of L. reuteri* supernatant (glycerol sol.) could be recognized.

The group treated with *L. reuteri* lysate alone was also confirmed to have somewhat lymphocyte-proliferative activity, although this activity was less than that of a reference stimulant.

### Example 3: Administration of L.reuteri in MCT oil to human young subjects to study the reduction of eczema.

This research was performed to determine whether simple dietary supplementation with *L. reuteri* and MCT oil can affect atopic eczema in young children currently under standard treatment. Case studies at the Children's Hospital in Lund have indicated that at least one child with persistent eczema that did not respond to standard therapy could be resolved by the administration of a dietary supplement *of L. reuteri* and MCT oil for 30 days. The present study defines that supplementation of standard therapy with *L. reuteri* can be confirmed to have such an effect under controlled study conditions.

The primary end point of this research is the reduction in the extent and severity of eczema according to a validated scoring system (SCORAD) due to administration *of L. reuteri* and MCT oil during a 12-month treatment period. Scores at the start of treatment are normalized. A reduction in the score in the *L. reuteri-*MCT group which is significantly greater than that in the placebo group at 3, 6, 9 and/or 12 months of treatment is considered the primary end point. This is an ongoing consecutive double blind randomized study. Patients aged between 3 months and 3 years of age (i.e. up to but not including the 4th birthday) are included after a diagnosis of moderate atopic dermatitis is made and after informed consent by the parent. The diagnosis need not be new, i.e. children already under standard treatment may be included.

Patients are recruited from a patient population who is seeing an allergy doctor. The investigators provides the parents of the potential participants with information about the study and if the inclusion and exclusion criteria are fulfilled and written consent is obtained, the patient is included. Patients are randomized into two cohorts, each containing 20 subjects. One group receives *L. reuteri* supplementation in MCT oil and the other a blinded placebo formulation containing only MCT oil. The Study Products are administered for 12 months.

The investigators fill out an eczema score form based on the examination of the patient and the eczema is photographed. The familial situation is also recorded according to a questionnaire to determine risk factors in the home environment. The Study Product is given to the parents and daily administration begins.

Both groups continue with their prescribed regular pharmaceutical treatment of the eczema. To measure the use of steroids the parents of the patients are asked to bring all their prescribed steroids with them to the hospital and the packages are weighed and recorded.

The parents of the patients are contacted by telephone 1 month after receiving the Study Product to monitor progress and the patients return to the clinic for full examination, photographic documentation of the eczema and a renewed eczema score form completion after 3, 6, 9 and 12 months. The 12 month completes the treatment.

Blood samples are taken at the start of the study to determine the levels of total IgE and the gm allotype of the patient and again at the completion of the study to determine total IgE levels. A skin prick test for egg, milk, fish, cat and peanut is performed at both time points.

The Study Product containers are returned, weighed and recorded to ensure compliance.

### Dosage

*L. reuteri* in MCT oil is given at a dose of 1 x 10⁸ CFU/day which is equivalent to 10 drops of the *L.reuteri-MCT* oil throughout the study. In the placebo group an equivalent amount of the identical MCT oil is given.

### Medications/interventions/diet

All forms of steroid are allowed and a specially modified diet if needed. The patients should be totally free from exposure of all other sorts of foods with added Lactobacilli and probiotics during the study.

### Definitions

The severity and extent of eczema is defined according to The SCORAD Index (Kunz B, Oranje AP, Labreze L, Stalder JF, Ring J, Taieb A..Dermatology. 1997;195(1):10-9.Clinical validation and guidelines for the SCORAD index: consensus report of the European Task Force on Atopic Dermatitis).

### Procedures at each visit

The study requires a total of 5 visits to the clinic by the participants with their parents and one telephone interview with a parent.

### First visit (Day 0)

After written consent is obtained, the patient is included in the study. The familial situation of the patient is recorded according to a questionnaire to determine allergy risk factors in the home environment. The investigator fills out an eczema score form based on the examination of the patient and the eczema is photographed. A blood sample is taken for analysis of Total IgE as well as gm allotype (genetic determinant for allergy). A skin prick test is performed to determine allergic reaction to egg, milk, fish, peanut and cat.

Each patient receives a randomization number and the corresponding Study Product (2 bottles) is given to the parents and daily administration begins. All patients continue with their prescribed regular pharmaceutical treatment of the eczema.

To measure the use of steroids the parents of the patients are asked to bring all their prescribed steroids with them to the hospital and the packages are weighed and recorded.

### Telephone interview (1 month)

The parents of the patients are contacted by telephone 1 month after the first visit to monitor progress, ensure that the Study Product is being administered and identify any problems the parents/patients may have in complying with the study protocol.

### Second visit (3 months)

The patients return to the clinic for full examination, photographic documentation of the eczema and completion of a renewed eczema score form. The parents of the patients are asked to bring all their prescribed steroids with them to the hospital and the packages are weighed and recorded. The first 2 Study Product bottles are weighed and recorded. One bottle should now be empty and this is retained by the investigators. The other should be partially empty and this is retained for continued use in the study. A further two Study Product bottles are supplied to the parents at this visit.

### Third visit (6 months)

The patients return to the clinic for full examination, photographic documentation of the eczema and completion of a renewed eczema score form. The parents of the patients are asked to bring all their prescribed steroids with them to the hospital and the packages are weighed and recorded. Study Product bottles are weighed and recorded. Two bottles should now be empty and these are retained by the investigators. The third bottle should be almost full and this is retained for continued use in the study. A further two Study Product bottles are supplied to the parents at this visit.

### Fourth visit (9 months)

The patients return to the clinic for full examination, photographic documentation of the eczema and completion of a renewed eczema score form. The parents of the patients are asked to bring all their prescribed steroids with them to the hospital and the packages are weighed and recorded. Study Product bottles are weighed and recorded. One bottle should now be empty and this is retained by the investigators. The second bottle should be half empty and the third bottle full. These two bottles should be retained for continued use in the study.

### Fifth and final visit (12 months)

The patients return to the clinic for a final full examination, photographic documentation of the eczema and completion of the final eczema score form. A blood sample is taken for analysis of total IgE. A skin prick test is performed to determine allergic reaction to egg, milk, fish, peanut and cat.

The parents should bring all prescribed steroids with them to the hospital and the packages are weighed and use recorded. Study Product bottles are weighed and recorded. The remaining two bottles should now be empty and these are retained by the investigators.

### Statistical methods and determination of sample size

The patients are randomized in two cohorts, each containing 25 subjects. Generally for each study, based on prior results, assuming a 50% effect of the treatment (as in earlier studies) and 80% power, 20 evaluable children per group should be sufficient to show an effect. Five extra children per group are included to cover drop-outs.

### Summary of results and discussion

**Table 3: Eczema score at 0, 3, 6, 9 and 12 months.**

| **L.reuteri ATCC 55730 + MCT-oil groupoil group** | | | | | |
|---|---|---|---|---|---|
| **Rand nr** | **0 month** | **3 month** | **6 month** | **9 month** | **12 month** |
| **2** | 58 | 0 | 16 | 37 | 36 |
| **6** | 34 | 6 | 0 | 0 | 0 |
| **7** | 48 | 6 | 4 | 0 | 4 |
| **10** | 35 | 7 | 0 | 8 | 7 |
| **11** | 34 | 7 | 0 | 0 | 4 |
| **12** | 30 | 2 | 1 | 1 | 4 |
| **15** | 50 | 14 | 8 | 12 | 10 |
| **17** | 28 | 11 | 4 | 10 | 11 |
| **19** | 26 | 0 | 0 | 0 | 0 |
| **21** | 28 | 10 | 20 | 13 | 4 |
| **23** | 23 | 10 | 8 | 4 | 8 |
| **25** | 26 | 4 | 4 | 4 | 4 |
| **27** | 31 | 13 | 27 | 18 | 10 |
| **29** | 44 | 19 | 17 | 14 | 13 |
| **30** | 56 | 18 | 38 | 18 | 18 |
| **31** | 44 | 4 | 19 | 11 | 10 |
| **35** | 48 | 14 | 14 | 7 | 20 |
| **36** | 28 | 6 | 4 | 4 | 4 |
| **39** | 44 | 4 | 0 | 0 | 0 |
| **40** | 35 | 4 | 0 | 4 | 4 |
| **AVG** | **37.50** | **7.95** | **9.20** | **8.25** | **8.55** |

| **MCT-oil group** | | | | | |
|---|---|---|---|---|---|
| **1** | 41 | 40 | 18 | 29 | 20 |
| **3** | 38 | 11 | 16 | 15 | 17 |
| **4** | 28 | 22 | 12 | 12 | 39 |
| **5** | 34 | 18 | 4 | 12 | 12 |
| **8** | 19 | 14 | 21 | 10 | 18 |
| **9** | 68 | 68 | 60 | 51 | 60 |
| **13** | 54 | 48 | 29 | 26 | 48 |
| **14** | 26 | 60 | 42 | 42 | 48 |
| **16** | 56 | 8 | 64 | 48 | 31 |
| **18** | 54 | 30 | 48 | 31 | 54 |
| **20** | 12 | 12 | 4 | 12 | 13 |
| **22** | 22 | 0 | 0 | 0 | 0 |
| **24** | 30 | 10 | 31 | 48 | 34 |
| **26** | 34 | 13 | 22 | 11 | 18 |
| **28** | 56 | 4 | 7 | 10 | 11 |
| **32** | 39 | 32 | 30 | 28 | 18 |
| **33** | 28 | 31 | 22 | 22 | 31 |
| **34** | 31 | 48 | 31 | 42 | 34 |
| **37** | 35 | 31 | 22 | 22 | 12 |
| **38** | 48 | 30 | 31 | 18 | 28 |
| **AVG** | **37.65** | **26.50** | **25.70** | **24.45** | **27.30** |

The combination of L.reuteri strain ATCC 55230, which is a good producer of 3HPA, with MCT oil gives an important improvement of the eczema.

### Example 4 Manufacturing of products containing selected strain

In this example, a product containing 3-HPA producing *Lactobacillus* and MCT is manufactured. The product preferably is an oil-based formulation containing *L. reuteri* SD2112 made for good stability and shelf life.

### Description of the manufacturing process

A flowchart of the preferred manufacturing process is shown in **Figure 5****.** Details of one such possible process that may be used in the invention herein is as follows.

### Mixing of ingredients

1. Mix the medium-chain triglyceride for example Akomed R, (Karlshamns AB, Karshamn, Sweden) with silicondioxide, Cab-o-sil M5P, (M5P, Cabot) in a Bolz mixing mashine tank (Manufacturer Alfred BOLZ Apparatebau GmbH, Wangen im Allgäu, Germany)
2. Homogenization. A Sine pump and dispax (Sine Pump, Arvada, Colorado, United States) are connected to the Bolz mixer and the mixture is homogenized.
3. Vacuum-drying. The mixture is dried under 10 mBar vacuum in the Bolz tank, for 12 hours to remove any water in the oil for extended shelflife of the product.
4. Adding *Lactobacillus reuteri.* About 20 kg of dried oil mixture is moved to a 50 liter stainless steel vessel. *L. reuteri* powder, for example, freeze dried cells, in an amount that would vary depending on amount wanted, is suspended in oil. For example, 0.2 kg of culture with activity of 10¹¹ CFU per g is added. It is mixed slowly until homogenous.
5. Mixing. The premix with *L. reuteri* is brought back to the Bolz mixer.
6. Discharging. The suspension is discharged to a 200 liter glass vessel, and covered with nitrogen. The suspension is held in the vessel until filling in product bottles.

The product as formulated herein may be use for oral administration. Alternatively, it may be formulated for tube-feeding to be added to an enteral nutrition product as is known in the art, or for topical application to the skin using standard components as known in the art to make it suitable for topical use.

While certain representative embodiments have been set forth herein, those skilled in the art will readily appreciate that modifications can be made without departing from the spirit or scope of the invention.

## Claims

1. A product for 3-HPA (reuterin) production in mammals, including humans, comprising 3-HPA producing *Lactobacillus reuteri* in combination with medium chain triglyceride oil.

2. The product of claim 1, wherein the strain of *Lactobacillus reuteri* is strain ATCC 55730.

3. The product of claim 1, wherein the medium chain triglyceride oil is Akomed R.

4. The product of claim 1, wherein the product is formulated for oral administration.

5. The product of claim 1, wherein the product is formulated for tube-feeding for addition to an enteral nutrition product.

6. The product of claim 1, wherein the product is formulated for topical application to the skin.

7. A method for 3-HPA (reuterin) productionin mammals, including humans, comprising administering to the mammal 3-HPA producing *Lactobacillus reuteri* in combination with medium chain triglyceride oil.

8. The method of claim 7, wherein the product is administered orally.

9. The method of claim 7, wherein the product is applied to the skin.
